# EUROPEAN PATENT SPECIFICATION

(11) **EP 2 340 723 B1**
(45) Date of publication and mention of the grant of the patent: **11.06.2014**
(21) Application number: 09816270.4
(22) Date of filing: 18.09.2009
(51) Int. Cl.: C12N 9/26, C12N 9/10, A23L 1/105, A23L 1/182

(54) **METHOD FOR PRODUCING COOKED RICE FOOD, AND ENZYME PREPARATION FOR IMPROVING COOKED RICE FOOD**
VERFAHREN ZUR HERSTELLUNG EINES GEKOCHTEN REISPRODUKTS UND ENZYMPRÄPARAT FÜR EIN VERBESSERTES GEKOCHTES REISPRODUKT
PROCÉDÉ POUR PRODUIRE UN ALIMENT DE RIZ CUIT, ET PRÉPARATION D ENZYME POUR AMÉLIORER UN ALIMENT DE RIZ CUIT

(30) Priority: 25.09.2008 JP 2008246481
(43) Date of publication of application: 06.07.2011
(73) Proprietor: Ajinomoto Co., Inc., Tokyo 104-8315 (JP)
(72) Inventor: YAMADA, Noriaki, Kawasaki-shi Kanagawa 210-8681 (JP); SATO, Hiroaki, Kawasaki-shi Kanagawa 210-8681 (JP); KODERA, Tomohiro, Kawasaki-shi Kanagawa 210-8681 (JP)
(74) Representative: Fischer, Heinrich
(86) International application number: PCT/JP2009/066875
(87) International publication number: WO 2010/035858

(56) References cited:
- EP-A2- 0 963 704
- WO-A1-2005/096839
- WO-A1-2008/001940
- WO-A1-2008/059992
- JP-A- 5 015 324
- JP-A- 5 316 974
- JP-A- 6 153 827
- JP-A- 7 147 917
- JP-A- 7 289 186
- JP-A- 9 206 006
- JP-A- 2004 041 046

## Description

### Technical Field

This invention relates to a method for producing a cooked rice food and an enzyme preparation for improving a cooked rice food, in which transglutaminase and α-glucosidase are used together with glucose oxidase.

### Background Art

Many foods are composed of various components such as starch, proteins, saccharides and lipids which combinedly constitute the textures of the foods. Among all, starch and proteins largely contribute to food texture and changes of starch with the passage of time are considered as particularly important.

When allowed to stand at an ordinary or low temperature, gelatinized starch liberates water and thus hardens. A number of studies have been made on this phenomenon that is called retrogradation of starch. To prevent retrogradation, it is generally required to maintain starch at a temperature of 80°C or higher, quickly dry starch to reduce the water content thereof to 15% or less, maintain starch in an alkaline state of pH 13 or higher, etc. Commonly known methods for preventing retrogradation comprise adding a saccharide (glucose, fructose, liquid sugar, etc.), soybean protein, wheat gluten, a fatty acid ester or a polysaccharide (yam, konjac, etc.) to starch-containing foods. For example, JP-A-59-2664 discloses a method which comprises adding a thickener, a surfactant, etc. However, these methods cause significant taste changes and can exert only unstable effects and, therefore, the aforesaid problem cannot be sufficiently solved thereby.

As a means for preventing retrogradation, it is also known to add enzymes. For example, JP-A-58-86050 discloses a method for improving cooked rice by adding an enzyme such asamylase, protease or lipase to polished rice, further adding sodium chloride and cyclodextrin thereto and then cooking the obtained mixture. JP-A-60-199355 discloses a method for preventing cooked rice from retrogradation by spraying cooked rice with an aqueous solution of saccharifying amylase (β-amylase, glucoamylase). Although attempts have been made to improve the qualities of cooked rice by adding various enzyme preparations to rice as discussed above, no remarkable effect has not been established so far.

According to WO2005/096839, soft and sticky cooked rice that is hardly degraded with the passage of time can be obtained by adding, as a quality-improving agent for starch-containing foods, transglucosidase being α-glucosidase to rice in the cooking step. Although this agent is effective to a certain degree, there still remain some points to be improved, i.e., loosening rice grains sticking together and improving the properties. Thus, it is very difficult by any of these methods to achieve both improvements in the texture of cooked rice at the point of the finish of cooking and maintenance of the thus improved texture for a long period of time.

Relating to the application of transglutaminase to cooked rice, there has been disclosed a method for producing cooked rice, which suffers from no degradation in flavor even in the case of storing a long time after cooking and to which stickiness is particularly added, by adding transglutaminase, a partially hydrolyzed protein, an oligosaccharide and a sugar alcohol to rice cooking water and then cooking. According to this disclosure, although the cooked rice prepared by adding transglutaminase alone has preferred crispness, it is inferior in stickiness and swallowing feeling to the cooked rice prepared by adding transglutaminase together with partially hydrolyzed wheat protein (JP-A-9-206006). Moreover, Japanese Patent No. 3310081 discloses a method for preventing degradation of starch in cooked rice with increased water content, during storage or distribution at a low temperature, by adding transglutaminase. However, the texture of rice grains in such cooked rice with increased water content is largely different from the texture of common cooked rice.

Since cooked rice contains a large amount of glucose, glucose oxidase likely exerts some effect thereon. However, it has never been reported to use this enzyme for improving the properties of cooked rice. Japanese Patent No. 3718495 discloses a method for producing a diet food comprising cooked rice by reducing the glucose content of cooked rice with the use of glucose oxidase. Japanese Patent No. 2808060 discloses a method for preventing degradation, that is caused by the oxidation of lipids by dissolved oxygen incorporated into glucose, by adding glucose oxidase and glucose in the production of a retort cooked rice food. However, these documents refer nothing to improvement in physical properties. Furthermore, there has never been reported so far, not only for improving physical properties but also for any other purpose, to use glucose oxidase together with α-glucosidase and/or transglutaminase in a cooked rice food. A number of reports have been made on the use of glucose oxidase in breads and it is known that glucose oxidase has an effect of improving bread dough stability. However, cooked rice is a food that is completely different from bread and, therefore, the effect achieved by adding glucose oxidase to cooked rice would largely duffer from those achieved by adding the same to bread.

### Disclosure of the Invention

The purpose of the present invention is to provide a method for producing a cooked rice food which has improved physical properties and palatability and an enzyme preparation for improving a cooked rice food. In particular, the purpose of the invention is to provide a method for improving the qualities (palatability and physical properties) of a cooked rice food immediately after producing, preventing degradation in the qualities thereof with the passage of time during the production process and the distribution process after the production, and, furthermore, improving the processing suitability of processed cooked rice. More specifically speaking, the invention aims at providing a method for producing a cooked rice food having such a texture including, for example, "glutinousness", "puffiness", "graininess" and "non-clumping properties" that cannot be obtained by using α-glucosidase alone, transglutaminase alone or a combination of these two enzymes,.

As the results of intensive studies, the present inventors have found out that the aforesaid purposes can be achieved by using transglutaminase and α-glucosidase. together with glucose oxidase, thus completing the invention. Namely, the invention is as follows.
[1] A method for producing a cooked rice food, which comprises using transglutaminase and α-glucosidase together with glucose oxidase.
[2] The method for producing a cooked rice food according to [1] above, which comprises treating rice with 0.0001 to 120 U of transglutaminase per g of uncooked rice as a raw material, and 0.03 to 300, 000 U of α-glucosidase per g of uncooked rice as a raw material, together with 0.001 to 500 U of glucose oxidase per g of uncooked rice as a raw material.
[3] The method for producing a cooked rice food according to [1] or [2] above, which comprises treating rice with 0.05 to 12 U of transglutaminase per g of uncooked rice as a raw material, and 15 to 150,000 U of α-glucosidase per g of uncooked rice as a raw material, together with 0.03 to 210 U of glucose oxidase per g of uncooked rice as a raw material.
[4] The method according to any one of [1] to [3] above, wherein glucose oxidase is added at a ratio of 0.003 to 10,000 U per U of transglutaminase and 0.000003 to 34 U per U of α-glucosidase.
[5] The method according to any of [1] to [4] above, wherein glucose oxidase is added at a ratio of 0.1 to 900 U per U of transglutaminase and 0.000006 to 3 U per U of α-glucosidase.
[6] An enzyme preparation for improving a cooked rice food, which comprises, as the active ingredients, transglutaminase and α-glucosidase together with glucose oxidase.
[7] The enzyme preparation according to [6] above, wherein the glucose oxidase content is at a ratio of 0.003 to 10,000 U per U of transglutaminase and 0.000003 to 34 U per U of α-glucosidase.
[8] The enzyme preparation according to [6] or [7] above, wherein the glucose oxidase content is at a ratio of 0.1 to 900 U per U of transglutaminase and 0.000006 to 3 U per U of α-glucosidase.

The invention makes it possible to improve a cooked rice food. In particular, the qualities (physical properties such as puffiness, glutinousness, graininess and easy loosening properties) of a cooked rice food immediately after producing can be improved and the degradation in the qualities thereof with the passage of time can be prevented.

In the method for producing a cooked rice food and the enzyme preparation for improving a cooked rice food according to the present invention, transglutaminase and α-glucosidase are used with glucose oxidase.

Glucose oxidase is an oxidizing enzyme catalyzing a reaction whereby gluconic acid and hydrogen peroxide are formed from glucose, oxygen and water that are employed as the substrates. Hydrogen peroxide that is formed by the reaction oxidizes an SH group in a protein and thus promotes the formation of an SS bond (a disulfide bond) to thereby form a cross-linked structure in the protein. There have been known glucose oxidases originating in various sources such as microorganisms, plants and so on. In the invention, any enzyme having the aforesaid activity can be used regardless of its origin. Also, use can be made of a recombinant enzyme. As an example thereof, a microorganism-origin glucose oxidase available from Shinnihon Chemicals Co. under the trade name "SUMIZYME PGO" may be cited. There have been marketed glucose oxidase preparations also containing catalase. These mixtures of glucose oxidase with other enzymes are also usable in the invention so long as having the glucose oxidase activity.

In the invention, transglutaminase means an enzyme having an activity of catalyzing a transacylation reaction wherein a glutamine residue serves as a donor and a lysine residue serves as a receptor in a protein or peptide. There have been known transglutaminases originating in various sources such as mammals, fishes, microorganisms and so on. In the invention, any enzyme having the aforesaid activity can be used regardless of its origin. Also, use can be made of a recombinant enzyme. As an example thereof, an actinomycete-origin transglutaminase available from Ajinomoto Co. under the trade name "ACTIVA TG" may be cited.

In the invention, α-glucosidase means an enzyme hydrolyzing an α-1,4-glucoside bond at the non-reducing end to form α-glucose. Among α-glucosidases, transglucosidase having a transglycosylating activity from an α-1,4 bond to an α-1,6 bond is preferred. As an example of α-glucosidase, the enzyme available from Amano Enzyme Inc. under the trade name transglucosidase L "AMANO" may be cited.

Example of the cooked rice food according to the invention include cooked rice (plain white rice), vinegar-seasoned rice (*sushi meshi*), adzuki bean rice, pilaf, fried rice, rice cooked with seasoned ingredients, cooked glutinous rice, rice gruel, risotto, rice ball, *sushi*, box lunch and so on. Furthermore, the invention includes in its scope products produced by freezing, aseptic-packing, retort pouch-packing, drying or canning these foods.

The rice to be used as the raw material of the cooked rice food according to the invention may be of any variety. Either soft rice or hard rice may be used and either newly harvested rice or old rice may be used. Either low-quality rice or high-quality rice may be used. Furthermore, use may be made of processed rice having been treated with an acid or an enzyme such as low-protein rice (protein content-adjusted rice).

In the method for producing the cooked rice food according to the invention, the treatment of rice with transglutaminase and α-glucosidase together with glucose oxidase may be conducted at any stage prior to cooking or after cooking. Namely, the enzymes may be added to soaking water in which rice is to be soaked to absorb water. Alternatively, the enzymes may be added at a point between soaking and cooking. It is also possible that, after cooking, the enzymes are sprinkled on cooked rice. In a production line for a steamed rice food, the enzymes may be sprinkled on rice before or after steaming. For treating rice, transglutaminase, α-glucosidase and glucose oxidase may be used in an arbitrary order without restriction. The treatment with either one or two enzymes may be first conducted followed by the treatment with the residual one (s) . In the case of using all of these three enzymes, however, it is preferred to treat rice with these three enzymes simultaneously. In addition, enzymes other than the aforesaid ones or other substances may be used together.

In the invention, glucose oxidase is added to treat rice at a ratio, in terms of enzyme activity, of 0.001 U or above, preferably 0.001 to 500 U and more preferably 0.03 to 210 U, per g of uncooked rice as a raw material. The treatment of rice with glucose oxidase exerts an effect of imparting remarkable "glutinousness" and "elasticity" to the cooked rice food. In particular, such "glutinousness" that cannot be obtained by adding transglutaminase or α-glucosidase can be achieved thereby. Although "elasticity" at a certain degree can be obtained by using transglutaminase and α-glucosidase, the "elasticity" can be improved by increasing the ratio of transglutaminase. In this case, however, the effect of α-glucosidase of imparting "puffiness" is somewhat reduced. By adding glucose oxidase, in contrast thereto, strong "elasticity" can be imparted without undesirably inhibiting the function of α-glucosidase. When 500 U or more glucose oxidase is added, the "elasticity" is excessively elevated and the resultant cooked rice food shows an unnatural gummy texture and, therefore, becomes less preferable.

The enzyme activity of glucose oxidase is determined by treating glucose, as a substrate, with glucose oxidase in the presence of oxygen to form hydrogen peroxide, treating the thus formed hydrogen peroxide with peroxidase in the presence of aminoantipyrine and phenol, and measuring the color tone of the quinone imine coloring matter thus formed at a wavelength of 500 nm. One unit (U) of the enzyme is defined as the amount of the enzyme required for oxidizing 1 µmol of glucose in 1 minute.

In the invention, when glucose oxidase is added at a ratio, in terms of enzyme activity, of 0.001 U or above, preferably 0.001 to 500 U and more preferably 0.03 to 210 U, per g of uncooked rice, transglutaminase is added to treat rice at a ratio, in terms of enzyme activity, of 0.0001 U or above, preferably 0.0001 to 120 U and more preferably 0.05 to 12 U, per g of uncooked rice. It is also desirable that transglutaminase is added in such an amount as to control the amount of added glucose oxidase to 0. 003 to 10, 000 U, preferably 0.1 to 900 U, per U of transglutaminase.

The enzyme activity of transglutaminase is determined by reacting benzyloxycarbonyl-L-glutaminyl glycine and hydroxylamine that are employed as substrates, forming an iron complex of the thus formed hydroxamic acid in the presence of trichloroacetic acid, measuring the absorbance at 525 nm, and then determining the amount of hydroxamic acid from a calibration curve to thereby calculate the activity. One unit (U) of the enzyme is defined as the amount of the enzyme required for forming 1. µmol of hydroxamic acid in 1 minute at 37°C and pH 6.0.

In the invention, α-glucosidase is added to treat rice at a ratio, in terms of enzyme activity, of 0.03 U or above, preferably 0.03 to 300, 000 U and more preferably 15 to 150, 000 U, per g of uncooked rice as a raw material. It is also desirable that α-glucosidase is added in such an amount as controlling the amount of added glucose oxidase to 0.000003 to 34 U, preferably 0.000006 to 3 U, per U of α-glucosidase.

One unit (U) of α-glucosidase is defined as the amount of the enzyme required for forming 1 µg of glucose in 2.5 ml of a liquid reaction mixture, in the case of adding 1 ml of a 0.02 M acetate buffer (pH 5.0) to 1 ml of 1 mM α-methyl-D-glucoside, adding 0.5 ml of an enzyme solution thereto and then reacting at 40°C for 60 minutes.

As stated above, in the case of producing a cooked rice food by treating rice with transglutaminase and α-glucosidase together with glucose oxidase, it is appropriate to add the enzymes at the ratios, in terms of enzyme activity (U), 0. 003 to 10,000 U of glucose oxidase, preferably 0.1 to 900 U of glucose oxidase per U of transglutaminase, and 0.000003 to 34 U, preferably 0.000006 to 3 U of glucose oxidase per U of α-glucosidase. When these enzymes are added respectively in the amounts as defined above, it is possible to produce a cooked rice food which has excellent qualities (i.e., properties such as puffiness, glutinousness, graininess and easy loosening properties) immediately after the production and suffers from less degradation in the qualities with the passage of time.

The reaction time of each enzyme is not particularly restricted so long as the enzymatic treatment of the substrate can be completed therein. Although an extremely short or long reaction time may be employed, it is preferred from a practical viewpoint to conduct the treatment for 5 minutes to 24 hours. Similarly, the reaction temperature may be arbitrarily selected so long as the enzyme can maintain the activity thereof. From a practical viewpoint, it is preferred to carry out the treatment at 0 to 80°C. That is, a sufficient reaction time can be obtained by a method commonly employed for cooking rice.

An enzyme preparation for improving a cooked rice food can be obtained by blending transglutaminase and α-glucosidase and glucose oxidase with food additives, for example, a bulking agent such as dextrin, starch, a processed starch or reducing maltose, a seasoning such as a meat extract, a protein such as a vegetable protein, gluten, egg albumen, gelatin or casein, a hydrolyzed protein, a partially hydrolyzed protein, an emulsifier, a chelating agent such as a citric acid salt or a polyphosphoric acid salt, a reducing agent such as glutathione or cysteine, alginic acid, alkaline water, fat or oil, a dye, a souring agent, a flavor and so on. The enzyme preparation according to the invention may be in any form such as a liquid, a paste, granules or a powder. The contents of the individual enzymes in the enzyme preparation are each more than 0% and less than 100% It is desirable that the glucose oxidase content is 0.003 to 10,000 U, preferably 0.1 to 900 U per U of transglutaminase, and 0.000003 to 34 U, preferably 0.000006 to 3 U per U of α-glucosidase.

### Brief Description of the Drawing

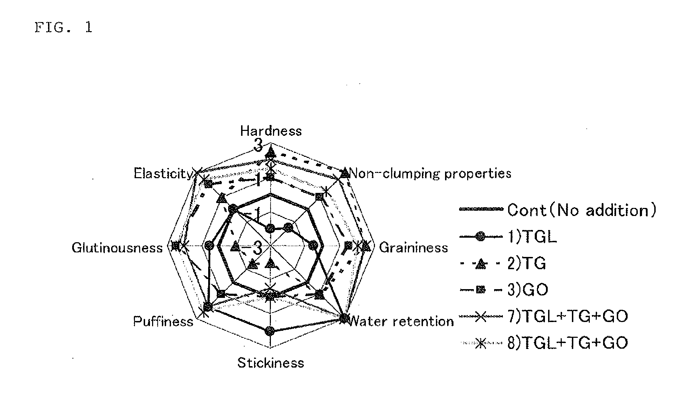

Fig. 1 shows the results of a sensory evaluation of frozen cooked rice (Example 1).

### Best Mode for Carrying Out the Invention

To illustrate the invention in greater detail, the following Examples will be given. However, it is to be understood that the invention is not restricted thereto.

### Example 1

300 g of uncooked rice "*Hokkaido Kirara 397*" was washed with tap water and soaked in tap water for one hour. After draining, the rice was supplied into an IH jar rice cooker Model NJ-HS06-S (manufactured by Mitsubishi Electric Co.). Next, tap water was added thereto so as to give a water amount 1.5 times as much as the uncooked rice amount. Namely, tap water was added in such an amount that the sum of the water absorbed by the uncooked rice during soaking and the amount of water added after soaking attained 450 g, i.e., 1.5 times as much as the amount of the uncooked rice (300 g). Then, "Transglucosidase L" (manufactured by Amano Enzyme Inc.; hereinafter referred to as TGL) that is α-glucosidase having a transglycosylating activity of converting an α-1,4 bond to an α-1, 6 bond, a transglutaminase preparation "ACTIVA TG" (manufactured by Ajinomoto Co., hereinafter referred to as TG) and a glucose oxidase preparation "SUMIZYME PGO" (hereinafter referred to as GO) were added thereto and dissolved therein. The obtained mixture was cooked with the aforesaid rice cooker. Table 1. shows the amounts of the individual enzymes added.

**Table 1: Amount of enzymes added in cooking rice**

| | Amount of preparation added | | | Addition ratio oil preparation | |
|---|---|---|---|---|---|
| | TGL (U/g of uncooked rice) | TG (U/g of uncooked rice) | GO (U/g of uncooked rice) | GO per TGL1U (U) | GO per TG 1U (U) |
| Cont (no addition) | - | - | - | - | - |
| 1) TGL | 70.69 | - | - | - | - |
| 2) TG | - | 0.26 | - | - | - |
| 3) GO | - | - | 0.95 | - | - |
| 4) TGL+TG | 70.69 | 0.26 | - | - | - |
| 5) TGL+GO | 70.69 | - | 0.95 | 0.013 | - |
| 6) TG+GO | - | 0.26 | 0.95 | - | 3.65 |
| 7) TGL+TG+GO | 70.69 | 0.26 | 0.95 | 0.013 | 3.65 |
| 8) TGL+TG+GO | 70.69 | 0.17 | 1.68 | 0.024 | 9.68 |

Each cooked rice food was quickly frozen at -40°C in a quick freezer "Blast Chiller/Shock Freezer Model QXF'-012SF5" (manufactured by Fukushima Industries, Co.) to give a frozen cooked rice. After storing for one week at -20°C, the frozen cooked rice was microwaved and sensorily evaluated. Table 2 shows the results. The sensory evaluation was conducted in eight items including hardness, glutinousness, water-retention, puffiness, stickiness, non-clumping properties, elasticity and graininess. Moreover, preference in using Japanese rice dishes and preference in using Western/Chinese rice dishes were given as total evaluation. In the sensory evaluation items, "hardness" means the strength of a stress felt in grinding with the teeth, "glutinousness" means a glutinous texture felt around the teeth in chewing, "water-retention" means showing no water-release from inside in chewing, "puffiness" means a complex feature consisting of "softness" (the opposite to "hardness,"),"glutinousness" and "water-retention", "stickiness," means the adhesiveness of grain surface, "non-clumping properties" means that grains can be loosened in the mouth without sticking together, "elasticity" means the strength of a repulsion force, i.e., restoration, in chewing, and "graininess" means a complex feature consisting of "non-clumping properties" and "elasticity". Japanese rice dishes include plain white rice, rice mixed with seasoned ingredients, etc., while Western/ Chinese rice dishes include pilaf, fried rice, etc. Although the evaluation items should be well-balanced in both of Japanese rice dishes and Western/Chinese rice dishes, a texture with strong "glutinousness" and "puffiness" is preferred in Japanese rice dishes, while a texture with strong "elasticity" and "graininess" is preferred in Western/Chinese rice dishes. The evaluation was made by five panelists and scored in the -3 to +3 scale, referring a sample in a control lot, where no enzyme was added, as to 0. The total evaluation was made by calculating theoretical scores in test lots where two or three enzymes were used together, based on the results of test lots where TGL alone, TG alone and GO alone were respectively added. For example, the theoretical score of sample 7) to be used in Japanese rice dishes was calculated as follows. Since the score of the TGL (70.69 U/g)-alone lot was 1 and 70. 69 U/g of TGL was used in sample 7), the theoretical score of this lot was expressed in: 1 x 70.69 / 70.69 = 1. Since the score of the TG (0.26 U/g)-alone lot was 0 and 0.17 U/g of TG was used in sample 7), the theoretical score of this lot was expressed in: 0 x 0.17/0.26 = 0. Since the score of the GO (0.95 U/g)-alone lot was 0.5 and 1.68 U/g of GO was used in sample 7), the theoretical score of this lot was expressed in: 0.5 x 1.68/0.95 = 0.884. Thus, the theoretical score of sample 7) to be used in Japanese rice dishes was calculated as: 1 + 0 + 0.884 = 1.884. Therefore, "1.884" is a theoretical value of total evaluation using Japanese rice dishes. The theoretical value thus calculated ("1.884" in this case) is compared with the practical score ("3" in this case). When these scores are the same, it can be understood that the theoretical effect, i.e., an additive effect is established. When the practical score is larger than the theoretical score, synergistic effects are established. Since the practical score "3" exceeds the theoretical score "1.884" in the above case, it can be concluded that synergistic effects were established in this case.

As Table 2 and Fig. 1. show, the addition of TGL alone mainly imparted "puffiness", "stickiness" and "water-retention", the addition of TG alone mainly imparted "hardness", "non-clumping properties" and "graininess", the addition of GO alone mainly imparted "glutinousness" and "elasticity". Thus, it can be understood that all of the eight evaluation items can be controlled with the use of these three enzymes. In the case of using TGL and TG together, effects of imparting "hardness" and remarkable "water-retention" were observed but the effect of imparting "puffiness" was weak and no "glutinousness" was imparted. In the case of using TGL together with GO, only weak effects of imparting "hardness", "non-clumping properties" and "graininess" were observed but remarkable "glutinousness","water-retention" and "puffiness" were imparted, thereby giving a texture suitable for Japanese rice dishes. In the total evaluation, the practical scores exceeded the theoretical scores both in Japanese rice dishes and Western/Chinese rice dishes, which indicates that the combined use of TGL with GO exerted synergistic effects. In the case of using TG and GO together, no effect of imparting "puffiness" was observed but remarkable "hardness", "non-clumping properties", "elasticity" and "graininess" were imparted, thereby giving a texture suitable for Western/Chinese rice dishes such as pilaf and fried rice. In the total 1 evaluation, the practical score exceeded the theoretical score in Western/Chinese rice dishes, which indicates that the combined use of TG with GO exerted synergistic effects. In 7) and 8) with the use of the three enzymes together, well-balanced effects were observed in each of the eight items. In the total evaluation, the practical scores of 7) and 8) both exceeded the theoretical scores both in Japanese rice dishes and Western/Chinese rice dishes, which indicates that the combined use of TGL and TG with GO exerted synergistic effects. With respect to "stickiness", the samples were cooked with water in an amount of giving sufficient "stickiness," and, therefore, no increase in "stickiness" by the addition of the enzymes is required any more. As discussed above, it was clarified that these three enzymes, i.e. TGL, TG and GO exert synergistic effects and give a preferable texture, when used in combination.

**Table 2: Sensory evaluation results of frozen cooked rice**

| | Sensory avaluation score (point) | | | | | | | | | | | | Theoretical score | |
|---|---|---|---|---|---|---|---|---|---|---|---|---|---|---|
| | Hard ness | Glutinousness | Water retention (no water liberation) | Puffi ness | Stickiness (surface) | Non clumping properties | Elasticity | Graini ness | Total evaluation (preference) | | | | Total evaluation (preference) | |
| | | | | | | | | | Japanese | | Western/ Chinese | | Japanese | Western/ Chinese |
| Cont (no addition) | 0 | 0 | 0 | 0 | 0 | 0 | 0 | 0 | 0 | × | 0 | × | - | - |
| 1) TGL | -2 | 0 5 | 3 | 2 | 2 | -1.5 | 0 | -0.5 | 1 | Δ | 0 | × | - | - |
| 2) TG | 2 5 | -1 | 1 | -1.5 | -2 | 3 | 1 | 2.5 | 0 | × | 0.5 | × | - | - |
| 3) GO | 1 | 2.5 | 1 | 1 | 0 | 1 | 2 | 1.5 | 0.5 | × | 0.5 | × | - | - |
| 4) TGL+TG | 2 | -0.5 | 3 | 0.5 | -0.5 | 1.5 | 1 | 1.5 | 1 | Δ | 1 | Δ | 1 | 0.5 |
| 5) TGL-GO | 0.5 | 3 | 3 | 3 | 2 | 0.5 | 1.5 | 1 | 2 | ○ | 1 | Δ | 1.5 | 0.5 |
| 6) TG+CO | 3 | 1.5 | 1.5 | 0 | -1.5 | 3 | 3 | 3 | 0.5 | × | 1.5 | ○ | 0.5 | 1 |
| 7) TGL+TG+GO | 2 | 2 | 3 | 2 | -0.5 | 2.5 | 3 | 2.5 | 2.5 | ○ | 3 | ○○ | 1.5 | 1 |
| 8) TGL+TG+GO | 1.5 | 2.5 | 3 | 2.5 | 0 | 1.5 | 2.5 | 2 | 3 | ○ | 2.5 | ○○ | 1.9 | 1.2 |

### Example 2

By using the enzymes, frozen cooked rice were obtained as in Example 1. Table 3 shows the ratios and amounts of the enzymes used. After storing for one week at -20°C, each frozen cooked rice was microwaved and sensorily evaluated. The sensory evaluation was made in eight items including hardness, glutinousness, water-retention, puffiness, stickiness, non-clumping properties, elasticity and graininess. Then, the preference of texture balance as plain white rice was expressed in the symbols ○○, ○, Δ and x. ○○ means "highly preferred", ○ means "preferred", Δ means "somewhat preferred" and x means "not preferred". The evaluation was made by five panelists. In Table 3, "3.4E+2", "5.6E-6" and so on indicate indexes and respectively mean "3.4x10²", "5.6x10⁻⁶" and the like.

**Table 3: Sensory evaluation results of frozen cooked rice**

| Test lot | 1 | 2 | 3 | 4 | 5 | 6 | 7 | 8 | 9 | 10 | 11 |
|---|---|---|---|---|---|---|---|---|---|---|---|
| TGL U/g of uncooked rice) | 0 | 0.003 | 0.03 | 15 | 38 | 45 | 50 | 75 | 88 | 95 | 100 |
| TG (U/g of uncooked rice) | 0 | 0.26 | 0.26 | 0.26 | 0.26 | 0.26 | 0.26 | 0.26 | 0.26 | 0.26 | 0.26 |
| GC (U/g of uncooked rice) | 0 | 0.84 | 0.84 | 0.84 | 0.84 | 0.84 | 0.84 | 0.84 | 0.84 | 0.84 | 0.84 |
| GO/TGL (ratio by U) | 0 | 3.4E+02 | 3.4E+01 | 5.6E-02 | 2.2E-02 | 1.9E-02 | 1.7E-02 | 1.1E-02 | 9.6E-03 | 8.8E-03 | 8.4E-03 |
| GO/TG (ratio by U) | 0 | 3.2E+00 | 3.2E+00 | 3.2E+00 | 3.2E+00 | 3.2E+00 | 3.22E+00 | 3.2E+00 | 3.2E+00 | 3.2E+00 | 3.2E+00 |
| Sensory evaluation result | × | × | Δ | ○ | ○○ | ○○ | ○○ | ○○ | ○○ | ○○ | ○○ |
| | | | | | | | | | | | |

| Test lot | 12 | 13 | 14 | 15 | 16 | 17 | 18 | 19 | 20 | 21 | 22 |
|---|---|---|---|---|---|---|---|---|---|---|---|
| TGL (U/g of uncooked rice) | 5000 | 150000 | 300000 | 375000 | 70.8 | 70.8 | 70.8 | 70.8 | 70.8 | 70.8 | 70.8 |
| TG (U/g of uncooked rice) | 0.26 | 0.26 | 0.26 | 0.26 | 0.00005 | 0.0001 | 0.05 | 0.14 | 0.16 | 0.18 | 0.23 |
| GO (U/g of uncooked rice) | 0.84 | 0.84 | 0.84 | 0.84 | 0.84 | 0.84 | 0.84 | 0.84 | 0.84 | 0.84 | 0.84 |
| GO/TGL (ratio by U) | 1.7E-04 | 5.6E-0E | 2.8E-06 | 2.2E-06 | 1.2E-02 | 1.2E-02 | 1.2E-02 | 1.2E-02 | 1.2E-02 | 1.2E-02 | 1.2E-02 |
| GO/TG (ratio by U) | 3.2E+00 | 3.2E+00 | 3.2E+00 | 3.2E+00 | 1.8E-04 | 9.1E+03 | 1.8E+01 | 6.1E+00 | 5.2E+00 | 4.6E+00 | 3.7E+00 |
| Sensory evaluation result | ○ | ○ | Δ | × | × | Δ | ○ | ○○ | ○○ | ○○ | ○○ |

| Test lot | 23 | 24 | 25 | 26 | 27 | 28 | 29 | 30 | 31 | 32 | 33 |
|---|---|---|---|---|---|---|---|---|---|---|---|
| TGL (U/g of uncooked rice) | 70.8 | 70.8 | 70.8 | 70.8 | 70.8 | 70.8 | 70.8 | 70.8 | 70.8 | 70.8 | 70.8 |
| TG (U/g of uncooked rice) | 0.32 | 0.35 | 0.37 | 0.41 | 11.5 | 115 | 230 | 0.26 | 0.26 | 0.26 | 0.26 |
| GO (U/g of uncooked rice) ¹ | 0.84 | 0.84 | 0.84 | 0.84 | 0.84 | 0.84 | 0.84 | 0.0001 | 0.001 | 0.03 | 0.42 |
| GO/TGL (ratio by U) | 1.2E-02 | 1.2E-02 | 1.2E-02 | 1.2E-02 | 1.2E-02 | 1.2E-02 | 1.2E-02 | 1.2E-06 | 1.2E-05 | 3.6E-04 | 5.9E-03 |
| GO/TG (ratio by U) | 2.6E+00 | 2.4E-00 | 2.3E+00 | 2.0E+00 | 7.3E-02 | 7.3E-03 | 3.7E-03 | 3.2E-04 | 3.2E-03 | 9.7E-02 | 1.6E+00 |
| Sensory evaluation result | ○○ | ○○ | ○○ | ○○ | ○ | Δ | × | × | Δ | ○ | ○○ |
| | | | | | | | | | | | |

| Test lot | 34 | 35 | 36 | 37 | 38 | 39 | 40 | 41 | 42 | 43 | 44 |
|---|---|---|---|---|---|---|---|---|---|---|---|
| TGL (U/g of uncooked rice) | 70.8 | 70.8 | 70.8 | 70.8 | 70.8 | 70.8 | 70.8 | 70.8 | 70.8 | 70.8 | 70.7 |
| TG (U/g of uncooked rice) | 0.26 | 0.26 | 0.26 | 0.25 | 0.26 | 0.26 | 0.26 | 0.26 | 0.26 | 0.26 | 0.17 |
| GO (U/g of uncooked rice) | 0.50 | 0.56 | 0.67 | 1.18 | 1.26 | 1.34 | 1.68 | 210 | 504 | 3360 | 1.68 |
| GO/TGL (ratio by U) | 7.1E-03 | 8.3E-03 | 9.5E-03 | 1.7E-02 | 1.8E-02 | 1.9E-02 | 2.4E-02 | 3.0E+00 | 7.1E-00 | 4.7E+01 | 2.4E-02 |
| GO/TG (ratio by U) | 1.9E+00 | 2.3E+00 | 2.6E+00 | 4.5E-00 | 4.8E+00 | 5.2E+00 | 6.5E-00 | 8.1E+02 | 1.9E+03 | 1.3E+04 | 9.7E+00 |
| Sensory evaluation result | ○○ | ○○ | ○○ | ○○ | ○○ | ○○ | ○○ | ○ | Δ | × | ○○ |

As Table 3 shows, when the three enzymes were used together, preferred effects were observed by adding TGL, TG and GO each in an amount falling within a specific range. Also, preferred effects were observed when the GO/TGL ratio (by U) and GO/TG ratio (by U) were each within a specific range. TGL exerted somewhat preferred effect at an addition ratio of 0.03 to 300,000 U and preferred effect at 15 to 150,000 U, each per g of uncooked rice. TG exerted somewhat preferred effect at an addition ratio of 0.0001 to 115 U and preferred effect at 0. 05 to 11.5 U, each per g of uncooked rice. GO exerted somewhat preferred effect at an addition ratio of 0.001 to 504 U and preferred effect at 0.03 to 210 U, each per g of uncooked rice. With respect to the ratio by U of GO to TGL, somewhat preferred effect was observed at 0.000003 to 34 U and preferred effect was observed at 0.000006 to 3 U. With respect to the ratio by U of GO to TG, somewhat preferred effect was observed at 0.003 to 9130 U and preferred effect was observed at 0.1 to 807 U. These results can be summarized as follows. In the case of adding TGL, TG and GO to a cooked rice food, it is preferable to add TGL in an amount of 0.03 to 300,000 U, more preferably 15 to 150,000 U, each per g of uncooked rice. It is preferable to add TG in an amount of 0.0001 to 120 U, more preferably 0.05 to 12 U, each per g of uncooked rice. It is preferable to add GO in an amount of 0.001 to 500 U, more preferably 0.03 to 270 U, each per g of uncooked rice. The ratio by U of GO to TGL is preferably 0.000003 to 34 U, more preferably 0.000006 to 3 U. The ratio by U of GO to TG is preferably 0.003 to 10,000 U, more preferably 0.1 to 900 U. By using the three enzymes together in the aforesaid ranges, preferable "hardness", "glutinousness", "water-retention", "puffiness", "stickiness", "non-clumping properties" and "graininess" can be achieved in a well-balanced manner without compromising any of these items. In particular, the simultaneous imparting of both a "hard" and "grainy" feel and a "glutinous" and "puffy" feel, which has been considered as difficult so far, contributes to the achievement of a preferable texture.

### Example 3

By using the enzymes, frozen cooked rice foods were obtained as in Example 1. The enzyme composition was fixed based on the composition (7 in Table 1) employed in Example 1 whereby preferable texture was obtained. The enzymes were added in the amounts listed in Table 9. After storing for one week at -20°C, each frozen cooked rice was microwaved and sensorily evaluated. The sensory evaluation was made in eight items including hardness, glutinousness, water-retention, puffiness, stickiness, non-clumping properties, elasticity and graininess. The preference as plain white rice was expressed in the symbols ○○,○,Δ and x. ○○ means "highly preferred", ○ means "preferred", Δ means "somewhat preferred" and x means "not preferred". The evaluation was made by four panelists.

**Table 4 Amounts of enzymes added in cooking rice**

| Test lot | Cont. | 1 | 2 | 3 | 4 | 5 | 6 | 7 | 8 | 9 | 10 |
|---|---|---|---|---|---|---|---|---|---|---|---|
| TGL (U/g of uncooked rice) | 0 | 0.071 | 0.71 | 7.07 | 70.69 | 1413.8 | 3534.5 | 7069 | 14138 | 35345 | 70690 |
| TG U/g of uncooked rice) | 0 | 0.00026 | 0.0026 | 0.026 | 0.26 | 5.20 | 13.0 | 26.0 | 52.0 | 130 | 260 |
| GO (U/g of uncooked rice) ¹ | 0 | 0.0010 | 0.010 | 0.10 | 0.95 | 19.0 | 47.50 | 95.0 | 190 | 475.0 | 950 |
| GO/TCL (ratio by U) | 0 | 0.013 | 0.013 | 0.013 | 0.013 | 0.013 | 0.013 | 0.013 | 0.013 | 0.013 | 0.013 |
| GO/TG (ratio by U) | 0 | 3.65 | 3.65 | 3.65 | 3.65 | 3.65 | 3.65 | 3.65 | 3.65 | 3.65 | 3.65 |
| Sensory evaluation result | × | × | Δ | ○ | ○○ | ○ | ○ | ○ | Δ | × | × |

As Table 4 shows, TGL exerted somewhat preferred effect at an addition ratio of 0.71 to 14,138 U and preferred effect at 7.07 to 7.069 U, each per g of uncooked rice. TG exerted somewhat preferred effect at an addition ratio of 0.0026 to 52 U and preferred effect at 0.026 to 26 U, each per g of uncooked rice. GO exerted somewhat preferred effect at an addition ratio of 0.01. to 190 U and preferred effect at 0.1 to 95 U, each per g of uncooked rice. When the enzymes were added in excessively large amounts, the strengthened hardness caused deterioration in texture balance. When the enzymes were added in excessively small amounts, the effects of the enzymes were limited and, therefore, no preferred effect was obtained.

### Example 4

300 g of uncooked rice "*Hokkaido Kirara 397*" was washed with tap water and soaked in tap water for one hour. After draining, the rice was supplied into an IH jar rice cooker Model NJ-HS06-S (manufactured by Mitsubishi Electric Co.). Next, tap water was added so as to give a water amount 1.5 times as much as the uncooked rice amount. Then, TGL, TG and GO were added thereto and dissolved, followed by cooking with the rice cooker. Table 5 shows the amounts of the enzymes. After adding a vinegar mixture in an amount of 10 wt% relative to the cooked rice thus obtained, the *sushi* rice thus obtained was molded in 25 g portions and quickly frozen at -40°C in a quick freezes "Blast Chiller/Shock Freezer Model QXF-012SF5" (manufactured by Fukushima Industries, Co.) to give a frozen *sushi* rice. The vinegar mixture comprised 63 of cereal vinegar, 31% of refined sucrose and 6% of sodium chloride. After storing for one week at -20°C, the frozen sushi rice was spontaneously thawed by allowing to stand for 3 hours at room temperature and then sensorily evaluated. Table 5 shows the results. The sensory evaluation was made in eight items including hardness, glutinousness, water-retention, puffiness, stickiness, non-clumping properties, elasticity and graininess. Moreover, preference in texture balance as *sushi* was expressed in the symbols ○○,○,Δ and ×. ○○ means "highly preferred", o means "preferred", Δ means "somewhat preferred" and x means "not preferred". The evaluation was made by four panelists

**Table 5. Amounts of enzymes added in cooking rice and sensory evaluation results of frozen sushi rice**

| Test lot | 1 | 2 | 3 | 4 | 5 | 6 | 7 | 8 |
|---|---|---|---|---|---|---|---|---|
| TGL (U/q of uncooked rice) | - | 312.50 | - | - | 70.69 | 70.69 | 141.38 | 147.38 |
| TG (U/g of uncooked rice) | - | - | 0.58 | - | 0.26 | 0.13 | 0.13 | - |
| GO (U/g of uncooked rice) ¹ | - | - | - | 1.05 | 0.95 | 0.95 | 0.95 | 0.95 |
| GO/TGL (ratio by U) | - | - | - | - | 0.013 | 0.013 | 0.007 | 0.007 |
| GO/TG (ratio by U) | - | - | - | - | 3.654 | 7.308 | 7.308 | - |
| Sensory evaluation result | × | × | × | × | ○○ | ○○ | ○○ | ○ |

As Table 5 shows, highly preferred effects were observed in the test lots where TGL, TG and GO were used together, and preferred effect was observed in the test lot where TGL and GO were used together, which suggests that the combined use of these enzymes enables the production of frozen *sushimeshi* having freeze resistance and a preferable texture.

### Example 5

25 g of uncooked rice "*Koshihikari*" was washed with tap water and drained for 60 minutes. Then, the rice was put into a retort pouch and tap water was added thereto to give a total weight including the uncooked rice of 252 g. After adding the enzymes, the pouch was sealed. Table 6 shows the amounts of the enzymes. After gently stirring and allowing to stand at room temperature for 60 minutes, the pouch was subjected to retort sterilization at 102°C for 10 minutes and then at 121°C until the F value attained 8 to give a retorted gruel. The retorted gruel was stored for two weeks at 20°C, then heated in boiling water for five minutes and sensorily evaluated. Table 6 shows the results. The sensory evaluation was made by paying attention to hardness, puffiness, graininess, glutinousness (viscosity), etc. and the preference of texture balance as gruel was expressed in the symbols ○○,○,Δ and ×. ○○ means "highly preferred", ○ means "preferred", Δ means "somewhat preferred" and × means "not preferred". The evaluation was made by five panelists.

**Table 6: Amounts of enzymes added in producing retort gruel and sensory evaluation results**

| Test lot | Test lot | 1 | 2 | 3 | 4 | 5 |
|---|---|---|---|---|---|---|
| TGL (U/g of uncooked rice) | TGL added | - | 312.50 | - | - | 70.69 |
| TG (U/g of uncooked rice) | TG added | - | - | 0.58 | - | 0.26 |
| GO (U/g of uncooked rice) | GO added | - | - | - | 1.05 | 0.95 |
| GO/TGL (ratio by U) | GO/TGL ratio by U | - | - | - | - | 0.013 |
| GO/TG (ratio by U) | GO/TG ratio by U | - | - | - | - | 3.654 |
| Sensory evaluation result | Sensory evaluation result | × | × | × | × | ○○ |

As Table 6 shows, highly preferred effect was observed in the test lot where TGL, TG and GO were used together, which suggests that the combined use of these enzymes enables the production of retort gruel having retort resistance and a preferable texture. Also, an effect of preventing the rice grains from breakage was observed.

### Example 6

A non-glutunous rice variety "*Hokkaido Kirara 397*" and a glutinous rice variety "*Mochigome*" (available from KITOKU-SHINRYO Co., Ltd.) were blended at each ratio listed in Table 7, washed with tap water and soaked in tap water for one hour. Then, the rice having been soaked was drained and supplied into an IH jar rice cooker Model NJ-H06-S (manufactured by Mitsubishi Electric Co.). Next, tap water was added so as to give a water amount 1.3 times or 1.45 times as much as the uncooked rice amount. Also, a retort-packed adzuki bean product for adzuki bean rice "*Osekihan no moto*" (manufactured by Imuraya Co., Ltd.) was added thereto. Further, TGL, TG and GO were added thereto and dissolved, followed by cooking with the aforesaid rice cooker. Table 7 shows the amounts of the enzymes. Each adzuki bean rice thus cooked was quickly frozen at -40°C in a quick freezer "Blast Chiller/Shock Freezer Model QXF-012SF5" (manufactured by Fukushima Industries, Co.) to give a frozen adzuki bean rice. After storing for one week at -20°C, the adzuki bean rice was microwaved and sensorily evaluated. Table 7 shows the results. The sensory evaluation was made in eight items including hardness, glutinousness, water-retention, puffiness, stickiness, non-clumping properties, elasticity and graininess. Moreover, preference in texture balance as adzuki bean rice was expressed in the symbols ○○, ○, Δ and ×. ○○ means "highly preferred", ○ means "preferred", Δ means "somewhat preferred" and × means "not preferred". The evaluation was made by four panelists. In a preferred texture as glutinous rice, it is seemingly highly important to have both glutinousness and stickiness.

**Table 7: Amounts of enzymes added in producing frozen adzuki bean rice and sensory evaluation results thereof**

| Test lot | 1 | 2 | 3 | 4 | 5 | 6 | 7 | 8 | 9 |
|---|---|---|---|---|---|---|---|---|---|
| Non-Glutinous rice (%) | 60 | 80 | 80 | 80 | 80 | 80 | 80 | 80 | 80 |
| Glutenous rice (%) | 40 | 20 | 20 | 20 | 20 | 20 | 20 | 20 | 20 |
| Water Addition ratio | 1.3 times | 1 45 times | 1.45 times | 1 45 times | 1.45 times | 1.45 times | 1.45 times | 1.45 times | 1.45 times |
| TGL (U/g of uncooked rice) | - | - | - | 62.50 | 187.50 | 312.50 | 312.50 | 70.69 | 70.69 |
| TG (U/g of uncooked rice) | - | - | - | - | - | - | - | 017 | 0.26 |
| GO (U/g of uncooked rice) ¹ | - | - | 1.68 | 1.68 | 1 68 | 1.68 | 2.52 | 1.68 | 0.95 |
| GO/TGL (ratio by U) | - | - | - | - | 0.009 | 0.005 | 0.008 | 0.024 | 0.013 |
| GO/TG (ratio by U) | - | - | - | - | - | - | - | 9.882 | 3.654 |
| Sensory evaluation result | ○○ | × | × | ○○ | ○○ | ○○ | ○○ | ○ | ○ |

As Table 7 shows, highly preferred effects were observed in the test lots where TGL and GO were used together, and highly preferred effects were observed in the test lot where TGL, TG and GO were used together, which suggests that the combined use of these enzymes enables the achievement of a preferred texture of adzuki bean rice even in the case of using glutinous rice at a lower ratio. Although the adzuki bean rice sample of test lot 1, wherein the glutinous rice was used at a high ratio, showed a preferred texture immediately after microwaving, i ts texture became gummy and hard after returning to room temperature. In contrast thereto, the adzuki bean rice samples of test lots 4 to 9, which had been treated with the enzymes, maintained preferred texture (sticky and glutinous) of adzuki bean rice after returning to room temperature. These results suggest that the use of the enzymes is also effective in preventing these products from deterioration with the passage of time.

### Example 7

Two non-Japanese rice varieties (produced in USA), i.e., "*SUSHI RICE*" (medium-grain size, available from Rice Tec) and "*Water Maid*" (long-grain size, available from RIVIANA FOODS) were washed with tap water and soaked in tap water for one hour. Then, the rice having been soaked was drained and supplied into an IH jar rice cooker Model NJ-HS06-S (manufactured by Mitsubishi Electric Co.). Next, tap water was added so as to give a water amount 1.6 times for the former or 1.9 times for the latter as much as the uncooked rice amount. Further, TGL, TG and GO were added thereto and dissolved, followed by cooking with the aforesaid rice cooker. Table 8 shows the amounts of the enzymes. Immediately after cooking, each cooked rice was sensorily evaluated. Table 8 shows the results. The sensory evaluation was made in eight items including hardness, glutinousness, water-retention, puffiness, stickiness, non-clumping properties, elasticity and graininess. Moreover, preference in texture balance as plain white rice was expressed in the symbols ○○,○,Δ and ×. ○○ means "highly preferred", ○ means "preferred", Δ means "somewhat preferred" and x means "not preferred". The evaluation was made by four panelists.

**Table 8: Amounts of enzymes added in cooking non-Japanese rice varieties and sensory evaluation results thereof**

| Test lot | 1 | 2 | 3 | 4 |
|---|---|---|---|---|
| Rice used | Medium grain size | | Long grain size | |
| water Addition ratio | 1.6 | | 1.9 | |
| TGL (U/g of uncooked rice) | - | 70.69 | - | 70.69 |
| TG (U/g of uncooked rice) | - | 0.26 | - | 0.26 |
| GO (U/g of uncooked rice) | - | 0.95 | - | 0.95 |
| GO/TGL (ratio by U) | - | 0.013 | - | 0 013 |
| GO/TG (ratio by U) | - | 3.654 | - | 3 654 |
| Sensory evaluation result | × | ○○ | × | ○ |

As Table 8 shows, preferred effects as plain white rice were observed in the test lots where TGL, TG and GO were used together, which suggests that the combined use oi these enzymes can also improve the qualities of foreign rice varieties, such as medium grain size and long grain size varieties, to thereby give preferred texture.

### Example 8

150 g of uncooked rice "*Hokkaido Kirara 397*" was washed with tap water and soaked in tap water for one hour. After draining, the rice was supplied into an IH jar rice cooker Model NJ-HS06-S (manufactured by Mitsubishi Electric Co.). Next, tap water was added so as to give a water amount 1.5 times as much as the uncooked rice amount. Then, TGL, TG and GO were added thereto and dissolved, followed by cooking with the rice cooker. Table 9 shows the amounts of the individual enzymes. Each cooked rice was fried in a frying pan using a seasoning blend for fried rice "*Rice Cook*" (manufactured by Ajinomoto Co.) for three minutes and then quickly frozen at -40°C in a quick freezer "Blast Chiller/Shock Freezer Model QXF-012SF5" (manufactured by Fukushima Industries, Co.) to give a frozen fried rice. After storing for one week at -20°C, the frozen fried rice was microwaved and then sensorily evaluated. Table 9 shows the results. The sensory evaluation was made in eight items including hardness, glutinousness, water-retention, puffiness, stickiness, non-clumping properties, elasticity and graininess. Moreover, preference in texture balance as fried rice was expressed in the symbols ○○, ○, Δ and ×. ○○ means "highly preferred", ○ means "preferred", Δ means "somewhat preferred" and × means "not preferred". The evaluation was made by three panelists.

As Table 9 shows, highly preferred effect was observed in the test lot where TGL, TG and GO were used together, which suggests that the combined use of these enzymes enables the production of fried rice having preferred texture.

**Table 9: Amounts of enzymes added in producing frozen fried rice and sensory evaluation results thereof**

| Test lot | 1 | 2 |
|---|---|---|
| TGL (U/g of uncooked rice) | - | 70.59 |
| TG (U/g of uncooked rice) | - | 0.26 |
| GO (U/g of uncooked rice) | - | 0.95 |
| GO/TGL (ratio by U) | - | 0.013 |
| GO/TG (ratio by U) | - | 3.654 |
| Sensory evaluation result | × | ○○ |

### Example 9

150 g of uncooked rice "*Hokkaido Kirara 397*" was washed with tap water and soaked in tap water for one hour. After draining, the rice was supplied into an IH jar rice cooker Model NJ-HS06-S (manufactured by Mitsubishi Electric Co.). Next, tap water was added so as to give a water amount 1.5 times as much as the uncooked rice amount. Also, a seasoning mix for seasoned rice "*Takikomi-tei*" for rice cooked with matsutake mushroom(manufactured by Ajinomoto Co.) was added thereto. Then, TGL, TG and GO were added thereto and dissolved, followed by cooking with the rice cooker. Table 10 shows the amounts of the individual enzymes. Each rice cooked with the seasoning mix was quickly frozen at -40°C in a quick freezer "Blast Chiller/Shock Freezer Model QXF-012SF5" (manufactured by Fukushima Industries, Co.) to give a frozen rice cooked with the seasoning mix. After storing for one week at -20°C:, the frozen rice cooked with the seasoning mix was microwaved and then sensorily evaluated. Table 10 shows the results. The sensory evaluation was made in eight items including hardness, glutinousness, water-retention, puffiness, stickiness, non-clumping properties, elasticity and graininess. Moreover, preference in texture balance as rice cooked with the seasoning mix was expressed in the symbols ○○, ○, Δ and ×. ○○ means "highly preferred", ○ means "preferred", Δ means "somewhat preferred" and × means "not preferred". The evaluation was made by three panelists.

**Table 10: Amounts of enzymes added in producing frozen rice cooked with the seasoning mix and sensory evaluation results thereof**

| Test lot | 1 | 2 |
|---|---|---|
| TGL (U/g of uncooked rice) | - | 70.69 |
| TG (U/g of uncooked rice) | - | 0.26 |
| GO (U/g of uncooked rice) | - | 0.95 |
| GO/TGL (ratio by U) | - | 0.013 |
| GO/TG (ratio by U) | - | 3.654 |
| Sensory evaluation result | × | ○○ |

As Table 10 shows, a highly preferred effect was observed in the test lot where TGL, TG and GO were used together, which suggests that the combined use of these enzymes enables the production of rice cooked with the seasoning mix having preferred texture.

### Industrial Applicability

Since the qualities of a cooked rice food can be improved according to the present invention, the invention is highly useful in the field of food industry.

## Claims

1. A method for producing a cooked rice food, which comprises using transglutaminase and α-glucosidase together with glucose oxidase.

2. The method for producing a cooked rice food according to claim 1, which comprises treating rice with 0.0001 to 120 U of transglutaminase per g of uncooked rice as a raw material, and 0.03 to 300, 000 U of α-glucosidase per g of uncooked rice as a raw material, together with 0.001 to 500 U of glucose oxidase per g of uncooked rice as a raw material.

3. The method for producing a cooked rice food according to claim 1 or 2, which comprises treating rice with 0.05 to 12 U of transglutaminase per g of uncooked rice as a raw material, and 15 to 150,000 U of α-glucosidase per g of uncooked rice as a raw material, together with 0.03 to 210 U of glucose oxidase per g of uncooked rice as a raw material.

4. The method according to any one of claims 1 to 3, wherein glucose oxidase is added at a ratio of 0.003 to 10,000 U per U of transglutaminase and 0.000003 to 34 U per U of α-glucosidase.

5. The method according to any of claims 1 to 4, wherein glucose oxidase is added at a ratio of 0.1 to 900 U per U of transglutaminase and 0.000006 to 3 U per U of α-glucosidase.

6. An enzyme preparation for improving a cooked rice food, which comprises, as the active ingredients, transglutaminase and α-glucosidase together with glucose oxidase.

7. The enzyme preparation according to claim 6, wherein the glucose oxidase content is at a ratio of 0.003 to 10,000 U per U of transglutaminase and 0.000003 to 34 U per U of α-glucosidase.

8. The enzyme preparation according to claim 6 or 7, wherein the glucose oxidase content is at a ratio of 0.1 to 900 U per U of transglutaminase and 0.000006 to 3 U per U of α-glucosidase.

## Patentansprüche

1. Verfahren zum Herstellen eines gekochten Reisnahrungsmittels, welches die Verwendung von Transglutaminase und α-Glucosidase zusammen mit Glucoseoxidase umfasst.

2. Verfahren zum Herstellen eines gekochten Reisnahrungsmittels nach Anspruch 1, welches das Behandeln von Reis mit 0,0001 bis 120 U Transglutaminase pro g ungekochten Reises als Ausgangsmaterial und 0,03 bis 300.000 U α-Glucosidase pro g ungekochten Reises als Ausgangsmaterial zusammen mit 0,001 bis 500 U Glucoseoxidase pro g ungekochten Reises als Ausgangsmaterial umfasst.

3. Verfahren zum Herstellen eines gekochten Reisnahrungsmittels nach Anspruch 1 oder 2, welches das Behandeln von Reis mit 0,05 bis 12 U Transglutaminase pro g ungekochten Reises als Ausgangsmaterial und 15 bis 150.000 U α-Glucosidase pro g ungekochten Reises als Ausgangsmaterial zusammen mit 0,03 bis 210 U Glucoseoxidase pro g ungekochten Reises als Ausgangsmaterial umfasst.

4. Verfahren nach einem der Ansprüche 1 bis 3, wobei Glucoseoxidase in einem Verhältnis von 0,003 bis 10.000 U pro U Transglutaminase und 0,000003 bis 34 U pro U α-Glucosidase zugegeben wird.

5. Verfahren nach einem der Ansprüche 1 bis 4, wobei Glucoseoxidase in einem Verhältnis von 0,1 bis 900 U pro U Transglutaminase und 0,000006 bis 3 U pro U α-Glucosidase zugegeben wird.

6. Enzymzubereitung zum Verbessern eines gekochten Reisnahrungsmittels, welches als aktive Bestandteile Transglutaminase und α-Glucosidase zusammen mit Glucoseoxidase umfasst.

7. Enzymzubereitung nach Anspruch 6, wobei der Glucoseoxidasegehalt in einem Verhältnis von 0,003 bis 10.000 U pro U Transglutaminase und 0,000003 bis 34 U pro U α-Glucosidase ist.

8. Enzymzubereitung nach Anspruch 6 oder 7, wobei der Glucoseoxidasegehalt in einem Verhältnis von 0,1 bis 900 U pro U Transglutaminase und 0,000006 bis 3 U pro U α-Glucosidase ist.

## Revendications

1. Procédé pour produire un aliment à base de riz cuit, qui comprend l'utilisation de transglutaminase et d'α-glucosidase avec de la glucose oxydase.

2. Procédé pour produire un aliment à base de riz cuit selon la revendication 1 qui comprend le traitement du riz avec 0,0001 à 120 U de transglutaminase par g de riz non cuit comme matière première, et 0,03 à 300 000 U d'α-glucosidase par g de riz non cuit comme matière première, avec 0,001 à 500 U de glucose oxydase par g de riz non cuit comme matière première.

3. Procédé pour produire un aliment à base de riz cuit selon la revendication 1 ou 2 qui comprend le traitement du riz avec 0,05 à 12 U de transglutaminase par g de riz non cuit comme matière première, et 15 à 150 000 U d'α-glucosidase par g de riz non cuit comme matière première, avec 0,03 à 210 U de glucose oxydase par g de riz non cuit comme matière première.

4. Procédé selon l'une quelconque des revendications 1 à 3, où la glucose oxydase est ajoutée à un rapport de 0,003 à 10 000 U par U de transglutaminase et 0,000003 à 34 U par U d'α-glucosidase.

5. Procédé selon l'une quelconque des revendications 1 à 4, où la glucose oxydase est ajoutée à un rapport de 0,1 à 900 U par U de transglutaminase et 0,000006 à 3 U par U d'α-glucosidase.

6. Préparation enzymatique pour améliorer un aliment à base de riz cuit qui comprend, comme ingrédients actifs, de la transglutaminase et de l'α-glucosidase avec de la glucose oxydase.

7. Préparation enzymatique selon la revendication 6, où la teneur en glucose oxydase est à un rapport de 0,003 à 10 000 U par U de transglutaminase et 0,000003 à 34 U par U d'α-glucosidase.

8. Préparation enzymatique selon la revendication 6 ou 7, où la teneur en glucose oxydase est à un rapport de 0,1 à 900 U par U de transglutaminase et 0,000006 à 3 U par U d'α-glucosidase.
